Europäisches Patentamt

(19) European Patent Office

Office européen des brevets

(11) EP 0 846 454 B1

(12) **EUROPEAN PATENT SPECIFICATION**

(45) Date of publication and mention
of the grant of the patent:
**02.11.2000 Bulletin 2000/44**

(51) Int Cl.⁷: **A61F 13/15**

(21) Application number: **96119391.9**

(22) Date of filing: **03.12.1996**

(54) **Absorbent articles having liquid shrinkable breathable backsheets**

Absorbierende Artikel mit bei Flüssigkeitskontakt schrumpfenden, atmungsaktiven unteren Schichten

Articles absorbants à feuilles arrières perméables aux gaz et rétrécissables en présence de liquides

(84) Designated Contracting States:
**AT BE CH DE DK ES FI FR GB GR IE IT LI LU NL PT SE**

(43) Date of publication of application:
**10.06.1998 Bulletin 1998/24**

(73) Proprietor: **THE PROCTER & GAMBLE COMPANY Cincinnati, Ohio 45202 (US)**

(72) Inventors:
• **Corzani, Italo**
  **66100 Chieti (IT)**

• **Ciammaichella, Fabio**
  **65126 Pescara (IT)**

(74) Representative: **Hirsch, Uwe Thomas et al Procter & Gamble European Service GmbH, Sulzbacher Strasse 40-50 65824 Schwalbach am Taunus (DE)**

(56) References cited:
**EP-A- 0 220 741          EP-A- 0 710 472
US-A- 4 447 240          US-A- 4 478 971
US-A- 4 713 068          US-A- 4 834 733
US-A- 5 447 788**

EP 0 846 454 B1

## Description

Field of the Invention

The present invention relates to the provision of breathable backsheets for use in absorbent articles which are activated when contacted with liquids.

Background of the Invention

The primary consumer needs which underlie development in the absorbent article field, in particular catamenials is the provision of products providing both a high protection and comfort level.

One means for providing consumer comfort benefits in absorbent articles is by the provision of breathable products. Breathability has typically concentrated on the incorporation of so called 'breathable backsheets' in the absorbent articles. Commonly utilised breathable backsheets are microporous films and apertured formed films having directional fluid transfer as disclosed in for example US-A-4 591 523. Both these types of breathable backsheets are vapour permeable allowing gaseous exchange with the environment. This thereby allows for the evaporation of a portion of the fluid stored in the core and increases the circulation of air within the absorbent article. The latter is particularly beneficial as it reduces the sticky feeling experienced by many wearers during use, commonly associated with the presence of an apertured formed film or film like topsheet, particularly over extended periods of time. This is a result of topsheets designed to achieve a clean and dry appearance. These topsheets tend to be smooth thereby minimising the build up of fluid on the surface of the topsheet. However, these benefits are achieved at the expense of comfort, particularly under hot and humid conditions, when due to their smooth surface texture they tend to become sticky to the skin.

However, the main drawback associated with the use of breathable backsheets in absorbent articles is the negative effect on the protection level performance, by leakage known as wet through onto the users garment. Although, breathable backsheets in principle only allow the transfer of materials in the gaseous state, physical mechanisms such as extrusion, diffusion and capillary action may still occur and result in the transfer of the fluids from the absorbent core through the backsheet and onto the users garments. In particular, these mechanisms become more dominant if the product is utilised during physical exertion, or for heavy discharge loads or over extended periods of time. Thus, whilst the incorporation of breathable backsheets in absorbent articles is highly desirable from a comfort standpoint, since the primary role of a backsheet still remains the prevention of liquid leakage, such breathable backsheets cannot be satisfactorily incorporated into products.

The problem of wet through onto users garments due to the incorporation of such breathable backsheets in absorbent articles has indeed also been recognised in the art. Attempts to solve the problem have mainly resided in the use of multiple layer backsheets such as those illustrated in US-A-4 341 216. Similarly EP-A-710 471 discloses a breathable backsheet comprising an outer layer of a gas permeable, hydrophobic, polymeric fibrous fabric and an inner layer comprising an apertured formed film having directional fluid transport. The backsheet construction preferably has no liquid transport/wet through under certain specified test conditions. Also EP-A-710 472 discloses a breathable backsheet consisting of at least two breathable layers which are unattached to one another over the core area. The backsheet construction preferably has no liquid transport/wet through under certain specified test conditions.

US-A-4 713 068 discloses a breathable clothlike barrier for use as an outer cover for absorbent articles. The barrier comprises at least 2 layers, a first layer having a specified basis weight, fibre diameter and pore size and a second layer comprising a continuous film of polyvinyl alcohol having a specified thickness. The barrier also has a specified water vapour transmission rate and level of impermeability.

However, none of the above proposed solutions have been able to provide a fully satisfactory solution to the problem of breathable backsheet wet through under all conditions. Furthermore, another problem associated with the exemplified multi layer backsheets is an increase in total thickness of the product and a reduction in the flexibility, both of which result in a consumer noticeable reduction in product comfort.

An alternative proposed solution to the problem of breathable backsheet wet through relates to the improvement of the absorbent material such that little or no liquid comes into contact with the backsheet, thereby preventing wet through. This is typically achieved by increasing the amount of absorbent material in the article. However, this results in an absorbent article which is extremely thick which is highly undesirable from a consumer comfort standpoint. Hence, the absorbent article whilst having the required protection level and still maintaining some comfort benefits by the presence of the breathable backsheet, suffers from a lack of comfort from a different source, in this case the increased dimensions of the article.

In addition the above solution also results in a reduction in the flexibility of the article, particularly evident as an increase in the cross section stiffness. It is however also well established that in order to be comfortable for the wearer absorbent articles need to be cross sectionally flexible. It is believed that the more cross sectionally flexible an absorbent article is, the less will it be noticeable to the wearer. Thus flexibility is another highly desirable comfort requirement of modern absorbent articles.

EP-A-705 583 and EP-A-705 584 propose longitudinally flexible absorbent articles which are vapour permeable. However, the exemplified absorbent articles are typically very thin and do not address the absorbency capacity of the article or the problem of wet through.

US-A-5 447 778 discloses a porous nonwoven liquid activated barrier suitable for use in absorbent articles. The barrier includes a fibrous nonwoven web in which at least 50% of the fibres are prepared from a liquid swellable polymer which is not significantly soluble in water such as swellable polyvinyl alcohols. In the presence of water the polymers swell to an extent sufficient to substantially block the passage of liquid through the fibrous nonwoven web. However, from the data given in the patent, the passage of liquids is not substantially blocked and thus these webs do not eliminate the wet through problem such that it appears that the swellable polymers incorporated in a nonwoven application have a relatively low capability of closing the pores in the material.

EP-A-0 220 741 reveals an absorbent article comprising a liquid pervious top sheet, an absorbent core and a breathable backsheet, in which an additional water absorbing shrinkable material, separate from the backsheet, is utilised around the side edge portions of the article. The function of the shrinkable material is to improve the fit of the absorbent article around the weaver when it is exposed to liquid.

Consequently, as the incorporation of breathable backsheets in absorbent articles results in reduction of the protection level, further desirable product comfort modifications such as reducing the thickness of the product and improving the flexibility of the product which would further acerbate the problem may not be incorporated in the absorbent article. Thus, there exists a dichotomy in the means available to provide increased consumer comfort in absorbent products and acceptable protection levels.

It is therefore an objective of the present invention to provide an absorbent article having improved comfort, by the provision of breathability throughout the absorbent article which continues to maintain an acceptable level of protection.

It has now been found that this objective may be achieved by the provision of an article having a breathable backsheet which comprises at least one layer comprising a non soluble, liquid shrinkable material.

An advantage of the present invention is that the backsheet when in the dry state allows the transfer of vapour and preferably vapour and air and in its wet state after shrinkage maintains at least a degree of vapour permeability whilst preventing the transfer of liquids.

### Summary of the Invention

The present invention relates to absorbent articles such as sanitary napkins and panty liners which are breathable by the incorporation of a breathable backsheet and which have a reduced tendency to exhibit garment wet through. The backsheet comprises at least one vapour permeable, apertured layer comprising from 10% to 100% by weight of a non soluble, liquid shrinkable material such as polyvinyl alcohol. Upon contact with the liquid discharge the material shrinks thereby causing the apertures of the layer to close and hence prevent the passage of liquid through the backsheet onto the garment of the wearer.

### Detailed Description of the Invention

The absorbent article according to the present invention comprises as an essential component, a breathable backsheet. The backsheet primarily prevents the extrudes absorbed and contained in the absorbent structure from wetting articles that contact the absorbent product such as underpants, pants, pyjamas and undergarments thereby acting as a barrier to fluid transport. In addition however, the breathable backsheet of the present invention when dry permits the transfer of at least water vapour and preferably both water vapour and air through it and thus allows the circulation of gases into and out of the backsheet. In addition however the backsheet also preferably continues to allows the transfer of water vapour through it after wetting. The backsheet typically extends across the whole of the absorbent structure and can extend into and form part of or all sideflaps, side wrapping elements or wings.

According to the present invention the breathable backsheet comprises at least one layer comprising from 10 % to 100 %, preferably from 25 % to 100 %, more preferably from 50% to 100%, most preferably from 75 % to 100 % by weight of said layer of said backsheet of a non water soluble, liquid shrinkable material.

According to the present invention any known material which shrinks when in contact with liquid is suitable for incorporation in the backsheet. The term shrinks as used herein refers to a material exhibiting contraction when in contact with liquid in at least one direction i.e. in the x transverse direction, the y longitudinal direction or the z vertical direction or a material which contracts in any combination of said directions.

The term liquid as used herein refers to any fluid containing at least 75% by weight of water such as body fluids including urine, menstrual fluid and the like. The term non soluble as used herein refers to a material which is not significantly soluble at body temperature such that it can be utilised for the desired function.

According to the present invention each backsheet layer or layers comprising the shrinkable material shrink such that it contracts, in at least the x, y, or z direction, preferably in the x or y direction of said backsheet i.e. in the plane of the backsheet and most preferably in both the x and y directions of said backsheet. According to the test methods described hereafter said material shrinks by at least 5%, preferably at least 25%, most preferably at least 40% in at least one direction of said layer of said backsheet.

The incorporation of at least one layer comprising a shrinkable material in the breathable backsheet allows the passage of water vapour and preferably both water vapour and air through it. Thus, before use and whilst the backsheet remains dry, the absorbent article is at least permeable to water vapour. After contact with liquid, the material shrinks thereby tending to close the apertures of said layer of the backsheet. This thereby prevents the passage of macroscopic matter and liquids through the backsheet and onto the garments of the wearer. As a result of the shrinkage of the layer the air permeability of the layer and hence the backsheet overall is reduced and is typically negligible once the layer has become wet. However, an advantage of the present invention is that the passage of water vapour is not significantly hindered by the shrinking action of the material on contact with liquids and hence whilst the air permeability is reduced the backsheet and hence the absorbent article is still breathable even when wet.

Preferably the shrinkable material should not undergo any chemical or physical changes during shrinkage, such that the structural integrity of the layer is retained even after shrinkage such that typically, shrinkage does not cause any chemical or macroscopic physical change in the material itself.

Preferred breathable layers for use as backsheets herein are those having a high vapour and air exchange when dry and which preferably maintain a degree of vapour permeability once wet, whilst preventing air exchange. Hence, according to the present invention each layer of the backsheet comprising the shrinkable material has a vapour permeability when dry of at least 500 $g/(m^2.24hrs)$, preferably at least 700 $g/(m^2.24hrs)$, most preferably at least 1000 $g/(m^2.24hrs)$. According to a preferred embodiment of the present invention each shrinkable layer also has an air permeability when dry of at least 1000 $l/(m^2s)$, preferably at least 2000 $l/(m^2s)$, most preferably at least 3000 $l/(m^2s)$. Less preferably when said layer comprising the shrinkable material is in the form of a 2-dimensional microporous film, in the dry state this backsheet layer is only water vapour permeable. Furthermore, each shrinkable layer has a air permeability when wet as described herein after under test methods of less than 50 $l/(m^2s)$, preferably less than 30 $l/(m^2s)$, more preferably less than 10 $l/(m^2s)$ and most preferably not higher than 5 $l/(m^2s)$. Furthermore, each shrinkable layer has a vapour permeability after wetting as described in the test methods of more than 50 $g/(m^2.24hrs)$, preferably more than 100 $g/(m^2.24hrs)$, more preferably more than 200 $g/(m^2.24hrs)$, most preferably more than 250 $g/(m^2.24hrs)$.

The amount of fluid discharge or the so called garment wet through area of staining, that contacts the garment of the wearer of the absorbent product incorporating a breathable backsheet according to the present invention, should preferably be less than 1000 $mm^2$, more preferably less than 100 $mm^2$, most preferably less than 10 $mm^2$, as defined in the wet through test method described herein after under test methods.

According to the present invention suitable shrinkable materials for use herein include polymers such as polyvinyl alcohols having a hydrolysis level of at least 88 %, preferably at least 95 %, most preferably at least 98 %. The polyvinyl alcohols are prepared by hydrolysis of polyvinyl acetate. The term hydrolysis level as used herein refers to the percent, in moles, of substitution of the acetate groups by hydroxyl groups. The higher the hydrolysis level of the resultant polyvinyl alcohol, the higher the crystalline character of the resultant polymer. It is believed the crystallinity of the polyvinyl alcohol is directly linked to the ability of the polymer to shrink when in contact with liquids, such that a high crystallinity results in greater shrinkage. In addition, the crystallinity and hence the ability to shrink of the polyvinyl alcohols can be further improved not only by chemically increasing the degree of acetate substitution, but also by processing the polymer under conditions of adequate mechanical stretching. Typically this can be achieved during the process of transformation of the polymers into fibres and structures such as layers and films having the required mechanical properties. In particular, tension (stretching) is applied during the formation of the fibres or films. It is believed that this process increases the orientation of molecular chains and greatly enhances the crystallinity level of the polyvinyl alcohol.

It is believed that in the case of polyvinyl alcohols the shrinkage is caused by the partial destruction and/or plasticisation by the contact liquid which is principally water of the highly crystalline structure of the polyvinyl alcohol in the fibres which occurs at a molecular level. Because the crystal structure of the polyvinyl alcohol is formed under tension, the residual, unbalanced tension is retained within the material. When in contact with water, the crystals are plasticised and become weaker. As a result the internal tensions tend to be relaxed, allowing the material to recover at least part of it original pre-stretched dimensions. In this manner it is believed that the material exhibits shrinkage.

According to the present invention said layer may comprise a mixture of shrinkable materials exhibiting different levels of shrinkability with liquid, in addition to other non shrinkable components, provided that the shrinkable material is present at least 10% by weight of the layer and the shrinkability of said layer remains within the previously specified limits.

Hence, said layer of said breathable backsheet may further comprise up to 90% by weight, preferably up to 75 %, more preferably from up to 50 % and most preferably up to 25 % by weight, of said layer of non shrinkable component materials. However, in the most preferred embodiment the backsheet layer comprises 100% of the shrinkable material. Suitable non shrinkable component materials for use herein may be both naturally and synthetically derived. For example, embodiments wherein the shrinkable backsheet layer is a fibrous woven or nonwoven layer, said layer may include non shrinkable component materials such as nylon, polyester and cotton. These materials may be intimately mixed within the fibre of shrinkable material itself and/or woven, melt blown or extruded together with the shrinkable material. For embodiments wherein the shrinkable backsheet layer is an apertured film, these materials may be traditional components of polymeric films such as plasticisers for example glycerol, glycols, polyglycols, trimethylol propane, triethanolamine and other usual minor components of films such as stabilisers (antioxidants, anti-UV), pigments, and mineral fillers.

According to the present invention the breathable backsheet comprises at least one layer comprising said shrinkable material. Hence, the layer or layers comprising the shrinkable material such as a polyvinyl alcohol, can be present in any form such as woven, nonwoven, 2-dimensional microporous film, 2-Dimensional macroporous film, macroscopically expanded films or formed apertured films. Preferably, the shrinkable layer is a fibrous woven, or a fibrous nonwoven or a 2-dimensional microporous or macroporous apertured film. Backsheets according to the present invention having more than one layer comprising a shrinkable material may therefore be selected from any of the above mentioned forms. Preferred breathable backsheet may comprise mixtures of such forms of layers comprising said shrinkable material, such as the combination of a woven layer and an apertured film layer. The shrinkable material may thus be incorporated into any breathable backsheet layer and hence include all of the types described herein after under the additional non shrinkable material backsheet layers .

Preferably the shrinkable material is incorporated into a woven or apertured layer as it is believed that in these configurations, where said material is spatially ordered the ability of the material to shrink is enhanced.

According to an embodiment of the present invention the shrinkable material is formed into fibres prior to the production of a fibrous woven or nonwoven layer. Typically, the material in the fibrous form has a fibre diameter of from 5 micrometers to 300 micrometers, preferably from 10 micrometers to 250 micrometers, most preferably from 50 micrometers to 150 micrometers. In addition, the average interfibre pore diameter is preferably from 5 micrometers to 300 micrometers. The fibre preferably comprises at least 50%, preferably at least 75% by weight of the fibre of said shrinkable material.

According to an embodiment wherein said material is incorporated into an apertured formed film or a 2-dimensional porous film, the average pore diameter is from 0.5 micrometers to 500 micrometers.

According to the present invention said backsheet comprises at least one layer comprising said non soluble liquid shrinkable material. In addition, said backsheet may optionally comprise optional additional layers which are not shrinkable and hence do not contain shrinkable materials as defined herein. Preferably, said backsheet comprises at least two layers and most preferably said backsheet consists of three layers. The additional backsheet layers can be of any form such as polymeric woven, nonwoven or apertured films such as 2-Dimensional, planar micro and macroporous films, macroscopically expanded films and formed polymeric apertured films. Furthermore, said additional layers may be of any chemical nature, as described herein after and may be formed from synthetic or naturally derived sources or mixtures thereof.

According to the present invention suitable additional layers for use in the breathable backsheets herein are layers which are vapour permeable and more preferably both vapour and air permeable. Suitable layers may be fibrous wovens, fibrous nonwovens, apertured films or any mixtures or combinations thereof. Suitable vapour permeable layers include 2-Dimensional, planar micro and macro-porous films, macroscopically expanded films and formed apertured films. According to the present invention the apertures in said layer may be of any configuration, but are preferably spherical or oblong and may also be of varying dimensions. Typically, the apertures have an average diameter of from 5 micrometers to 500 micrometers. 2 dimensional planar porous films for use herein may have apertures having diameters from 200 micrometers to 5 micrometers. 2 dimensional planar microporous layers have apertures having average diameters of from 150 micrometers to 5 micrometers, preferably from 120 micrometers to 10 micrometers, most preferably from 90 micrometers to 15 micrometers. 2 dimensional planar macro porous layers have apertures having an average diameter of from 90 micrometers to 200 micrometers. Macroscopically expanded film layers and formed apertured layers have apertures having an average diameter of from 75 micrometers to 500 micrometers. The apertures preferably are evenly distributed across the entire surface of the layer, however layers having only certain regions of the surface having apertures are also envisioned.

Suitable 2-Dimensional planar layers of the backsheet may be made of any material known in the art, but are preferably manufactured from commonly available polymeric materials. Suitable materials are for example XMP-1001 of Minnesota Mining and Manufacturing Company, St. Paul, Minnesota, USA and Exxaire XBF-101W, supplied by the Exxon Chemical Company. As used herein the term 2-Dimensional planar layer refers to layers having a depth of less than 1mm, preferably less than 0.5mm, wherein the apertures have an average uniform diameter along their length and which do not protrude out of the plane of the layer. The apertured materials for use as a backsheet in the present invention may be produced using any of the methods known in the art such as described in EP-A-293 482 and the references therein.

Suitable apertured formed films include films which have discrete apertures which extend beyond the horizontal plane of the garment facing surface of the layer towards the core thereby forming protuberances. The protuberances have an orifice located at its terminating end. Preferably said protuberances are of a funnel shape, similar to those described in US 3, 929,135. The apertures located within the plane and the orifices located at the terminating end of protuberance themselves maybe circular or non circular provided the cross sectional dimension or area of the orifice at the termination of the protuberance is smaller than the cross sectional dimension or area of the aperture located within the garment facing surface of the layer. Preferably said apertured preformed films are uni directional such that they have at least substantially, if not complete one directional fluid transport towards the core.

Suitable macroscopically expanded films for use herein include films as described in for example in US 4 637 819 and US 4 591 523.

Preferably, said shrinkable material is incorporated into at least one of the backsheet layers. In multiple breathable backsheet embodiments wherein only one of the backsheet layers comprises the shrinkable material of the present invention, this layer should be positioned towards the absorbent core, preferably such that said layer does not form the garment facing surface of the absorbent article. Most preferably said layer is positioned such that it is in direct contact with the absorbent core.

According to a preferred embodiment of the present invention the backsheet comprises at least two layers, a first layer comprising said shrinkable material and a second layer comprising a non shrinkable polymeric layer comprising a fibrous woven, a fibrous nonwoven, a 2-Dimensional apertured porous film or a formed apertured film.

According to a most preferred embodiment of the present invention the backsheet consists of three layers, a first layer comprising said non soluble, liquid shrinkable material and a second layer comprising a polymeric apertured formed film and a third layer comprising a polymeric fibrous nonwoven.

According to a preferred embodiment of the present invention the breathable backsheet preferably has in the dry state, both air and water vapour permeability at the levels defined herein above for the shrinkable layers. More preferably, the breathable backsheet also meets the vapour permeability levels in the wet state.

According to the present invention the absorbent articles further comprise a topsheet and absorbent core. The absorbent material or core can be a fluffy fibrous absorbent core, comprising hydrogel particles if desired, or laminated tissues with or without particulate materials including hydrogel particles. The absorbent core fibres can be any of those known in the art including cellulose fibres or polymeric fibres rendered absorbent or even non absorbent matrix fibres. Also tissues of sufficient basis weight and absorbency can be used in the absorbent core according to the present invention.

According to the present invention the topsheet may comprise a single layer or a multiplicity of layers. In a preferred embodiment the topsheet comprises a first layer which provides the user facing surface of the topsheet and a second layer between the first layer and the absorbent structure/core. The topsheet provides a layer through which the liquids to be absorbed penetrate to the absorbent material.

The topsheet as a whole and hence each layer individually needs to be compliant, soft feeling, and non-irritating to the wearer's skin. It also can have elastic characteristics allowing it to be stretched in one or two directions. Typically, the topsheet extends across the whole of the absorbent structure and can extend into and form part of or all of the preferred sideflaps, side wrapping elements or wings. According to the present invention the topsheet may be formed from any of the materials available for this purpose and known in the art, such as non woven fabrics, films or combinations of both. In a preferred embodiment of the present invention at least one of the layers of the topsheet comprises a hydrophobic, liquid permeable apertured polymeric film. Preferably, the upper layer is provided by a film material having apertures which are provided to facilitate liquid transport from the wearer facing surface towards the absorbent structure, as detailed for example in US 3 929 135, US 4 151 240, US 4 319 868, US 4 324 426, US 4 343 314 and US 4 591 523.

According to the present invention the absorbent article is constructed by joining the various elements such as topsheet, backsheet and absorbent core by any means well known in the art. For example the backsheet and/ or topsheet may be joined to the absorbent core or to each other by a uniform continuous layer of adhesive, a patterned layer of adhesive, or an array of separate lines, spirals or spots of adhesive. Alternatively, the elements may be joined by heat bonds, pressure bonds, ultra sonic bonds, dynamic mechanical bonds or any other suitable joining means known in the art and any combination thereof.

Preferably the breathable backsheet is joined to other elements of the absorbent article so as to minimise and preferably eliminate any reduction in the vapour permeability of the backsheet.

In a preferred embodiment of the present invention the shrinkable layer of backsheet is only partially joined to any one of the additional components of the absorbent article, in order to allow the layer to freely contract when contacted with liquid and thereby prevent deformation of the article unless this is desired. One means of achieving this is for example to join the layer comprising the shrinkable material at only one point or along one line, alternatively the layer may be joined at more than one point or along two lines whereby the movement of the layer will be restricted. However such a movement restriction can be overcome by utilising at excess of the layer with respect to the other components of the article. Alternatively in another embodiment said layer is not joined to any other component of the absorbent article.

Preferably, in addition to the breathable backsheet the resultant absorbent article itself should also exhibit breathability. Hence the absorbent article preferably when dry has a vapour permeability of greater than 100 g/(m$^2$. 24hrs), preferably greater than 300 g/(m$^2$.24hrs), more preferably greater than 500 g/(m$^2$.24hrs) and most preferably greater than 700 g/(m$^2$.24hrs), as defined in the absorbent article vapour permeability test. More preferably the absorbent article also has an air permeability of greater than 100 l/(m$^2$s), preferably greater than 250 l/(m$^2$s), more preferably greater than 500 l/(m$^2$s), most preferably greater than 600 l/(m$^2$s), when dry, as defined in the absorbent article air permeability test.

According to the present invention the absorbent article may find utility as sanitary napkins, panty liners, adult incontinence products and baby diapers. The present invention finds particular susceptibility as sanitary napkins and panty liners. Thus, in addition to the components described herein above, the absorbent article may also comprise all those features and parts which are typical for products in the context of their intended use such as wings and side flaps, undergarment adhesive means, release paper, wrapping elements, fastening means and the like.

### Examples

Shrinkable layers

Example 1

This is a woven layer supplied by Nitivy Company, Japan under the code name Nitivy GF400 (code 4) containing 100% polyvinyl alcohol (PVOH) fibres wherein the warp fibres are PVOH fibre Solvron SH 28DEN -9FIL and the weft fibres are PVOH fibre Solvron SL 75DEN-20FIL. The layer has a basis weight of 43 g/m$^2$.

Example 2

This is a woven polyvinyl alcohol layer supplied by Nitivy, Japan under the code name Nitivy GFL400 (code SL) and contains 100% PVOH fibres fabric having warp fibres of PVOH fibre Solvron SL 28DEN -100FIL and weft of PVOH fibre Solvron SL 28DEN-96FIL. The layer has a basis weight of 28 g/m$^2$.

All PVOH Solvron SL and SH fibres contain more than 92.5 % of Polyvinyl Alcohols having a hydrolysis level of not less than 98 %.

Reference:

The reference is a commercially available 50 microns non porous polyvinyl alcohol film available under the code name Aquafilm Hot Water, supplied by LIN-PAC Co, UK.

Sanitary napkin examples

Example 1: Reference 1

This is an example of a sanitary napkin according to the present invention. The sanitary napkin is based on an Always Ultra sanitary napkin available from Procter & Gamble Germany which has been modified. The topsheet is a CPM material available from Tredegar Film Products B. V. Holland under the code X-1522. The core material is a tissue laminate (20 cm x 6.5 cm) composed of a 2 layers of airlayed tissue of 55 g/m$^2$ basis weight {available from Unikay Italy under the supplier code Unikay 303 LF}. Between the two tissue layers the laminate contains AGM (available from DOW Chemicals Germany under the supplier code; DOW 95890.1) at a basis weight of 51g/m$^2$. The core laminate was manufactured and supplied by Korma Italy (under the experimental manufacturing code: XA 07.001.003). The backsheet comprises two layers, a first layer in contact with the absorbent tissue, and a second layer, in contact with the wearers undergarment. The first layer is a formed apertured film backsheet layer which is a blend of low and high density PE with a crush resistant hexagonal hole configuration {supplied by Tredegar Film Products B.V. Holland under the manufacturing code AS 225 HD 25}. The second backsheet layer is a nonwoven laminate composed of two layers with basis weight 28g/m$^2$ (manufactured by Corovin GmbH in Germany under the trade name MD2005).

Example 2: Reference 2

This example is identical to example 1 except that the backsheet comprises three layers, a first layer in contact with the absorbent tissue, a middle layer and a third layer, in contact with the wearers undergarment and the middle layer. The first layer is a hydrophobic polyester spunlaced nonwoven (basis weight 40 g/m2) produced by Suominen Company, Finland. The second layer is a formed apertured film backsheet layer which is a blend of low and high density PE with a crush resistant hexagonal hole configuration {supplied by Tredegar Film Products B.V. Holland under the manufacturing code AS 225 HD 25}. The third backsheet layer is a nonwoven laminate composed of two layers with basis weight 28g/m$^2$ (manufactured by Corovin GmbH in Germany under the trade name MD2005).

Example 3

This example is identical to the reference 1 except that the backsheet comprises three layers, a first layer in contact with the absorbent tissue, a middle layer and a third layer, in contact with the wearers undergarment and the middle layer. The first layer is a woven supplied by Nitivy Company, Japan under the code name Nitivy GF400 (code 4) containing 100% PVOH fibres wherein the warp fibres are PVOH fibre Solvron SH 28DEN -9FIL and the weft fibres are PVOH fibre Solvron SL 75DEN-20FIL and having basis weight = 43 g/m$^2$. The middle layer is a formed apertured film backsheet layer which is a blend of low and high density PE with a crush resistant hexagonal hole configuration {supplied by Tredegar Film Products B.V. Holland under the manufacturing code AS 225 HD 25}. The third backsheet layer is a nonwoven laminate composed of two layers with basis weight 28g/m$^2$ (manufactured by Corovin GmbH in Germany under the trade name MD2005).

Example 4

This example is identical to example 3 except that the first layer of the backsheet has been replaced by a polyvinyl alcohol woven supplied by Nitivy, Japan under the code name Nitivy GFL400 (code SL) and is a 100% PVOH fibres fabric having warp fibres of PVOH fibre Solvron SL 28DEN -100FIL and weft of PVOH fibre Solvron SL 28DEN-96FIL and having basis weight of 28 g/m$^2$.

The initial dry dimensions of the PVOH layer in all the above exemplifications are identical to those of the absorbent core.

**Test methods**

Preparation of Test Fluid

The test liquid used herein is sheep's blood that has been modified by the addition of surfactant. The fluid closely resembles human menstrual fluid in viscosity, electrical conductivity, surface tension and appearance. The surfactant (1%) is introduced to the sheep's blood (Pegosperse 200ML, supplied by Pegesis/USA) to better reflect stress situations in which typical hygiene practice (and in some limited situations, dietary influences) may introduce additional surfactants or unexpected levels of, for example, fatty acids, that might lower the blood surface tension. Low surface tension menses is the biggest contributor to through backsheet wet-through failure on breathable absorbent articles such as sanitary napkins.

Reagents:

    1) Difibrinated sheep's blood, available from Unipath S.p.A {Garbagnate Milanese/Italy}.
    2) Surfactant, available from Pegesis, USA, as Pegosperse 200ML.

Firstly, the viscosity, pH and conductivity of the sheep's blood are measured to ensure the blood characteristics lie in a range close to that of normal menstrual blood {(see reference H.J. Bussing "zur Biochemie des Menstrualblutes" Zbl Gynaec, 179,456 (1957)}. The viscosity should lie in the range of 7 to 9 (units cStk). The pH should lie in the range of 6.9 to 7.5 and the conductivity in the range 10.5 to 13 (units mmho). If the viscosity is not within the range specified above, it should not be used and a new batch of sheep's blood needs to be obtained.

For individual measurements typically 100 ml sheep's blood with surfactant is prepared by mixing 99 ml sheep's blood (maintained at 25 °C) with 1 ml Surfactant. The sheep's blood/1% surfactant test fluid must be constantly mixed to ensure the components do not separate prior to usage. The sheep's blood should be used within 5 days from shipment from the supplier. The test fluid should be used within 4 hours of preparation.

**Wet through test**

Wet-Through is measured on the exemplified sanitary napkins as described below:

The test sanitary napkin is placed on a flat transparent test stand made of Poly-methyl-methacrylate (PMMA) or glass. The test sample is oriented such that the backsheet is in contact with the PMMA test stand. Suspended above the sample is a liquid delivery system that is capable of delivering any desired quantity of test liquid. Located between the test sample and the PMMA stand are two sheets of absorbent filter paper {produced by Cartiera Favini S. p. A., Italy; type Assorbente Bianca "N30" (local vendor Ditta Bragiola S.p.A., Italy)}. The test fluid is introduced to the test sample via a calibrated delivery system such as a burette. The loading is made by dripping at a uniform speed, 10 ml of test solution in 120 seconds in the centre of the test sanitary napkin. Once loaded a stationary plate is placed onto the sample which is then charged with weights so as to provide a uniform pressure of 60 g/cm$^2$ for a period of 5 minutes. The weight is then removed and the wet through on the absorbent filter paper assessed.

The wet through is determined by measuring the area of the eventual blood stain(s) on the blotter paper using a videocamera (DXC - 3000P, manufactured by Sony Co., Japan), to provide a photograph of the stained paper together with a 5 cm sample length. The stain area is calculated by processing the electronic photograph using Image Analysis software, Optimas 4.1, available from BioScan Inc, USA. The test is repeated at least 5 times.

Results

| Sanitary napkin example | Area of blood stain(s),mm$^2$ | |
| --- | --- | --- |
| | Minimum | Maximum |
| Reference 1 | 1515 | 2330 |
| Reference 2 | 1320 | 1420 |
| Example 3 | zero | 3 |
| Example 4 | zero | 5 |

The results are clearly demonstrative of the ability of the absorbent articles according to the present invention to effectively eliminate wet through onto the users garment as defined in the above test.

**Air & Vapour Permeability Test on absorbent article products and individual backsheet layers**

The Vapour permeability test is utilised to quantify the vapour transmission properties of breathable absorbent articles and individual backsheet layers.

Basic Principle of the Methods:

The basic principle of the test is to quantify the extent of water vapour transmission of a material or absorbent article. The test method that is applied is based on a standardized textile industry applied test method and commonly referred to as the "Upright cup test method". The test is performed in a stable temperature/humidity laboratory maintained at a temperature of 23° C at 50% RH for a period of 24 hours.

Apparatus:

  1) Sample cup (open area=0.00059 m2)
  2) Syringe to introduce the distilled water into the completed sample cup.
  3) Wax to seal the cup once sample has been arranged.
  4) A circular punch to facilitate preparation of circular samples of diameter = 30 mm.
  5) Laboratory of stable climatic conditions (23° C ± 0.5°C / RH = 50% ± 1 % RH)
  6) Laboratory balance accurate to 4 decimal places.

Sample Preparation / Measurements:

The test may be performed on the absorbent article product or an individual backsheet layer. A representative article is selected and a sample is cut to size using the punch. The sample cut is sufficiently large to adequately overlap the sample holder and to ensure material that may have been damaged or undesirably stretched due to the cutting operation lies outside of the measurement centre when the measurement is performed. The sample is so arranged onto of the sample cup so as to fully overlap the cup. The sample is oriented so as to ensure that the surface exposed to the laboratory environment is the same that would be found while wearing the article.

The closure ring of the sample cup is then placed onto the sample and pushed down. This ensures that the excess material is held firmly in place and does not interfere with the measurement. A wax is then applied to the entire surface of the closure ring to ensure the whole upper part of the apparatus is closed to the environment. Distilled water $(5 \pm 0.25$ ml) is introduced with the syringe into the sealed sample cup via the minute perforation. Finally this perforation is sealed with silicone grease.

The entire cup (containing sample and water) is weighed and the weight recorded to 4 decimal places. The cup is then placed in a ventilation stream generated by a fan. The air flowing over the top of the sample cup is $3 \pm 0.3$ m/sec and confirmed via a wind velocity meter ("Anemo", supplied by Deuta SpA., Italy). The sample cup remains in the ventilated test field for a period of 24 hrs and is then re-weighted. During this period if the test sample is sufficiently breathable the liquid in the sample holder is able to diffuse out of the sample holder and into the laboratory environment. This results in a reduction in the weight of water in the sample holder that can be quantified on re-weighing the complete sample cup following the 24 hr period.

The vapour permeability value is determined as the weight loss divided by the open area of the sample holder and quoted per day.
i.e.

$$\text{Vapour Permeability} = \text{Weight Loss (g)} / (0.00059 \text{ m}^2 / 24 \text{ hrs})$$

| Shrinkable layer examples | Vapour permeability g/(m$^2$.24hrs) |
|---|---|
| Example 1 | 470 |
| Example 2 | 280 |
| Reference | 480 |

From the above results, it can be concluded that the shrinkable layers retain a degree of water vapour permeability even after shrinkage. The vapour permeability value for the reference material demonstrates that film layers (polyvinyl alcohol) without any apertures still maintain a significant permeability to water vapour, due to their hydrophilic nature.

**Air Permeability test on absorbent article products and individual backsheet layers.**

The air permeability test is utilised to assess the ability of a material to circulation/exchange air.

Basic Principle of the Methods:

The basic principle of the test is to evaluate the resistance of a material or absorbent article to the passage of air. This test measures the volume (or amount) of air that flows through an article of given dimensions under standard conditions (of 23 °C /50% RH) and under a given difference of pressure. The instrument utilised for the test is: Air Permeabilimeter FX 3300 manufactured by TexTest AG Switzerland.

100mm square samples were prepared for air permeability measurements. For the measurements on dry samples, the samples are conditioned for 12 hours at 23 °C and 50 % of relative humidity. To measure the Air Permeability in the wet state, the samples have been wetted as described under the shrinkage test. The excess water is removed by gently pressing the samples on a blotter paper and the Air Permeability is measured within 10 minutes.

A square sample of 100mm dimensions is placed on the device as instructed by the manufacturer. An aspiration pump is used to generate a pressure of 1210 kPa that sucks air through the sample layer or structure. The device measures the volume of air flow at constant pressure drop across the orifices that contains the sample and measurement head. Finally the device generates a value of air permeability in the units of "l/(m$^2$s)".

In the case of vapour and air permeability measurements on a finished product the area of Panty Fastening Adhesive (PFA) can influence the results particularly if the adhesive is applied as is typically the case as an impervious stripe of adhesive via a slot coater. The measurements of both air and vapour permeability need to be representative of the total product. One simple way to ensure a representative measurement is to assess backsheet samples with some PFA coverage. In the exemplified sanitary napkins used herein the total backsheet area is covered to a degree of 45% with PFA. The vapour and air permeability measurements for sanitary napkins Reference 1 and 2 as well for Examples 3 and 4 are thus performed on the samples featuring a 45% surface coverage by PFA adhesive.

Results

The air permeability of the layer containing the shrinkable layer and for the sanitary napkins exemplified herein above were tested and the results are given below:

| Shrinkable layer | DRY l/(m$^2$s) | WET l/(m$^2$s) |
|---|---|---|
| Example 1 | 3250 | 5 |
| Example 2 | 9000 | 3 |

| Sanitary napkin example | DRY l/(m$^2$s) | WET l/(m$^2$s) |
|---|---|---|
| Reference 1 | 675 | 675 |
| Reference 2 | 550 | 550 |
| Example 3 | 530 | 44 |
| Example 4 | 545 | 35 |

From the above it can be seen that the air permeability of example 3 and 4 which are representative of the present invention are significantly reduced after wetting. Further testing of an additional sanitary napkin as described in Example 3 but wherein the shrinkable layer has been replaced by a monolithic commercial Polyethylene film, which is impermeable to both liquid and gases, gave a wet value of 32 (not included in the table). Hence it can be seen that even impermeable layers do not deliver zero air permeability, due to the difficulty in obtaining a perfect seal. Hence, the values obtained for example 3 and 4 effectively correspond to an air impermeable layer.

**Shrinkage test**

Samples of 100mm squares are cut from the shrinkable layer, and conditioned for 12 hours at 23 °C and 50 % relative humidity. The test is also carried out under these conditions. The sample is uniformly wetted using a pipette by 10 cm$^3$ of distilled water at 37°C. The sample dimensions are measured after 60 seconds. The percent shrinkage is expressed as:

$$\% \text{ shrinkage} = (\text{initial dimension} - \text{final dimension}) / (\text{initial dimension})* 100$$

Dimension is either the x, y or z length

| Shrinkable layer | Shrinkage in weft | Shrinkage in warp |
|---|---|---|
| Example 1 | 40% | 6% |
| Example 2 | 40% | 40% |

**Claims**

1. An absorbent article comprising a liquid pervious topsheet, an absorbent core and a breathable backsheet, characterised in that said backsheet comprises at least one layer comprising from 10% to 100 % of a non soluble, liquid shrinkable material.

2. An absorbent article according to claim 1, wherein said layer of said backsheet has a shrinkage of at least 5 % in one direction as defined in the shrinkage test.

3. An absorbent article according to claim 1, wherein said material is a polyvinyl alcohol having a hydrolysis level of at least 88%.

4. An absorbent article according to claim 3, wherein said polyvinyl alcohol has a hydrolysis level of at least 95%.

5. An absorbent article according to any one of the preceding claims, wherein said layer of said backsheet has an air permeability when dry of at least 1000 l/(m$^2$s) and an air permeability when wet of less than 50 l/(m$^2$s) as defined in the air permeability test.

6. An absorbent article according to any one of the preceding claims, wherein said layer of said backsheet, when wet has a water vapour permeability of at least 50 g/(m$^2$.24hrs) as defined in the vapour permeability test.

7. An absorbent article according to any one of the preceding claims, wherein said article has a wet through area of staining of less than 1000 mm$^2$ as defined in the wet through test.

8. An absorbent article according to any one of the preceding claims, wherein said article has a wet through area of staining of less than 100 mm$^2$ as defined in the wet through test.

9. An absorbent article according to any one of the preceding claims, wherein, said article has a vapour permeability when dry of at least 100 g/(m$^2$.24hrs) as defined in the vapour permeability test.

10. An absorbent article according to any one of the preceding claims, wherein said layer comprises a fibrous nonwoven or a fibrous woven or a 2-Dimensional microporous apertured film, or a 2-Dimensional macroporous apertured film.

11. An absorbent article according to any one of the proceeding claims, wherein said backsheet comprises at least two layers, a first layer comprising said shrinkable material and a second layer comprising a non shrinkable polymeric layer comprising a fibrous woven, a fibrous nonwoven, a 2-Dimensional apertured porous film or a polymeric apertured formed film.

12. An absorbent article according to any one of the preceding claims, wherein said backsheet consists of three layers, a first layer comprising said non soluble, liquid shrinkable material, a second layer comprising a polymeric apertured formed film and a third layer comprising a polymeric fibrous nonwoven.

13. An absorbent article according to any of the preceding claims, wherein said article is a sanitary napkin or a panty liner.

## Patentansprüche

1. Absorbierender Artikel mit einer flüssigkeitsdurchlässigen Oberschicht, einem absorbierenden Kern und einer atmungsfähigen Unterschicht, dadurch gekennzeichnet, daß die Oberschicht wenigstens eine Lage mit 10% bis 100% eines nicht löslichen, unter Flüssigkeit schrumpfenden Materials umfaßt.

2. Absorbierender Artikel nach Anspruch 1, in welchem die Lage der Unterschicht ein Schrumpfvermögen gemäß Schrumpfungstest von wenigstens 5% in einer Richtung hat.

3. Absorbierender Artikel nach Anspruch 1, in welchem das Material ein Polyvi nylalkohol mit einem Hydrolysegrad von wenigstens 88% ist.

4. Absorbierender Artikel nach Anspruch 3, in welchem der Polyvi nylalkohol einen Hydrolysegrad von wenigstens 95% hat.

5. Absorbierender Artikel nach einem der vorstehenden Ansprüche, in welchem die Lage der Unterschicht im trokkenen Zustand eine Luftdurchlässigkeit gemäß Luftdurchlässigkeitstest von wenigstens 1000 l/(m$^2$s) und im feuchten Zustand eine Luftdurchlässigkeit von wenigstens 50 l/(m$^2$s) hat.

6. Absorbierender Artikel nach einem der vorstehenden Ansprüche, in welchem die Lage der Unterschicht im nassen Zustand eine Wasserdampfdurchlässigkeit gemäß Dampfdurchlässigkeitstest von wenigstens 50 g/(m$^2$.24Std.)

hat.

**7.** Absorbierender Artikel nach einem der vorstehenden Ansprüche, in welchem der Artikel gemäß Durchnässungstest eine Durchnässungsfläche bei Beschmutzung von weniger als 1000 mm$^2$ hat.

**8.** Absorbierender Artikel nach einem der vorstehenden Ansprüche, in welchem der Artikel gemäß Durchnässungstest eine Durchnässungsfläche bei Beschmutzung von weniger als 100 mm$^2$ hat.

**9.** Absorbierender Artikel gemäß einem der vorstehenden Ansprüche, in welchem der Artikel im trockenen Zustand eine Dampfdurchlässigkeit gemäß Dampfdurchlässigkeitstest von wenigstens 100g/(m$^2$.24Std.) hat.

**10.** Absorbierender Artikel nach einem der vorstehenden Ansprüche, in welchem die Lage ein Faservlies oder ein Fasergewebe oder einen zweidimensionalen mikroporösen, mit Öffnungen versehenen Film oder einen zweidimensionalen makroporösen, mit Öffnungen versehenen Film umfaßt.

**11.** Absorbierender Artikel nach einem der vorstehenden Ansprüche, in welchem die Unterschicht wenigstens zwei Lagen umfaßt, wobei eine erste Lage das schrumpffähige Material umfaßt und eine zweite Lage eine nicht schrumpffähige Polymerschicht mit einem Fasergewebe, einem Faservlies, einem zweidimensionalen, mit Öffnungen versehenen porösen Film oder einen polymerisch offen geformten Film umfaßt.

**12.** Absorbierender Artikel nach einem der vorstehenden Ansprüche, in welchem die Unterschicht aus drei Lagen besteht, wobei eine erste Lage das nicht lösliche, unter Flüssigkeit schrumpffähige Material umfaßt, eine zweite Lage ein polymerisch offen geformten Film umfaßt und eine dritte Lage ein polymerisches Faservlies umfaßt.

**13.** Absorbierender Artikel nach einem der vorstehenden Ansprüche, in welchem der Artikel eine Damenbinde oder eine Höscheneinlage ist.

## Revendications

**1.** Article absorbant comprenant une feuille de dessus perméable aux liquides, une âme absorbante et une feuille de fond pouvant respirer, caractérisé en ce que ladite feuille de fond comprend au moins une couche comprenant de 10 % à 100 % d'un matériau non soluble, rétrécissable en contact avec des liquides.

**2.** Article absorbant selon la revendication 1, dans lequel ladite couche de ladite feuille de fond présente un rétrécissement d'au moins 5 % dans une direction, comme cela est défini dans l'essai de rétrécissement.

**3.** Article absorbant selon la revendication 1, dans lequel ledit matériau est un alcool polyvinylique ayant un niveau d'hydrolyse d'au moins 88 %.

**4.** Article absorbant selon la revendication 3, dans lequel ledit alcool polyvinylique a un niveau d'hydrolyse d'au moins 95 %.

**5.** Article absorbant selon l'une quelconque des revendications précédentes, dans lequel ladite couche de ladite feuille de fond a une perméabilité à l'air lorsqu'elle est sèche d'au moins 1000 l/(m$^2$s) et une perméabilité à l'air lorsqu'elle est humide inférieure à 50 l/(m$^2$s), comme cela est défini dans l'essai de perméabilité.

**6.** Article absorbant selon l'une quelconque des revendications précédentes, dans lequel ladite couche de ladite feuille de fond, lorsqu'elle est humide, a une perméabilité à la vapeur d'eau d'au moins 50 g/m$^2$.24h, comme cela est défini dans l'essai de perméabilité à la vapeur.

**7.** Article absorbant selon l'une quelconque des revendications précédentes, dans lequel ledit article a une zone de souillure à humidité traversante inférieure à 1000 mm$^2$, comme cela est défini dans l'essai d'humidité traversante.

**8.** Article absorbant selon l'une quelconque des revendications précédentes, dans lequel ledit article a une zone de souillure à humidité traversante inférieure à 100 mm$^2$, comme cela est défini dans l'essai d'humidité traversante.

**9.** Article absorbant selon l'une quelconque des revendications précédentes, dans lequel ledit article a une perméabilité à la vapeur lorsqu'il est sec d'au moins 100 g/(m$^2$.24h), comme cela est défini dans l'essai de perméabilité à la vapeur.

10. Article absorbant selon l'une quelconque des revendications précédentes, dans lequel ladite couche comprend un non-tissé fibreux ou un tissé fibreux ou un film microporeux perforé à 2 dimensions ou un film macroporeux perforé à 2 dimensions.

11. Article absorbant selon l'une quelconque des revendications précédentes, dans lequel ladite feuille de fond comprend au moins deux couches, une première couche comprenant ledit matériau rétrécissable et une deuxième couche comprenant une couche polymère non rétrécissable comprenant un tissé fibreux, un non-tissé fibreux, un film poreux perforé à 2 dimensions ou un film formé polymère perforé.

12. Article absorbant selon l'une quelconque des revendications précédentes, dans lequel ladite feuille de fond est constituée de trois couches, une première couche comprenant ledit matériau non soluble, rétrécissable en contact avec des liquides, une deuxième couche comprenant un film formé polymère perforé et une troisième couche comprenant un non-tissé fibreux polymère.

13. Article absorbant selon l'une quelconque des revendications précédentes, dans lequel ledit article est une serviette hygiénique ou une garniture de culotte.